# EUROPEAN PATENT APPLICATION

(11) **EP 4 796 643 A1**
(43) Date of publication of application: **26.08.2026**
(21) Application number: 24879200.4
(22) Date of filing: 01.10.2024
(51) Int. Cl.: C12P 7/62, C08G 63/02, C12M 1/02, C02F 11/02, C02F 11/13

(54) **METHOD AND DEVICES FOR PRODUCING FERTILISERS FOR AGRICULTURAL USE AND MICROBIAL BIOPLASTIC FROM ORGANIC WASTE**

(30) Priority: 18.10.2023 ES 202330860
(71) Applicant: QUÍMICA TÉCNICA ECOLÓGICA, S.L.U., 08221 Terrassa (Barcelona) (ES); Beda Water Engineering, S.L., 08860 Castelldefels (ES)
(72) Inventor: FERNÁNDEZ GARCÍA, Gustavo, 08860 Castelldefels, Barcelona (ES); VALERO HERNÁNDEZ, Antonio, 08860 Castelldefels, Barcelona (ES); MALERO TALAVERA, Sergio, 08221 Terrassa, Barcelona (ES)
(74) Representative: Isern Patentes y Marcas S.L.
(86) International application number: PCT/ES2024/070597
(87) International publication number: WO 2025/083303

(57) **Abstract**

The present invention relates to a method and devices for producing potassium- and nitrogen-based fertilisers, as well as bacterial bioplastic from plant and animal organic waste.

The present invention relates to a process and to the devices required for transforming plant and/or animal organic waste into various types of nitrogen-, potassium- and phosphorus-based fertilisers as well as PHA bacterial bioplastic.

According to the process disclosed herein, the residual organic matter generated by human activity will be used as raw material to generate, on the one hand, various types of nitrogen- and potassium-based fertilisers for agricultural use and, on the other hand, bacterial bioplastic.

## Description

### Object of the invention

The present invention, in accordance with the wording of this specification, relates to a process and to the devices required for transforming residual organic matter into various types of fertilisers for agriculture and bacterial bioplastic.

According to the process disclosed herein, the residual organic matter generated by human activity will be used as raw material to generate, on the one hand, nitrogen- and potassium-based fertilisers for agricultural use and, on the other hand, bacterial bioplastic.

### Background of the invention

On 22 March 2023, patent 2 936 907 was published with the title, METHOD AND DEVICES FOR PRODUCING POLYHYDROXYALKANOATES AND ROOT BIOSTIMULANT FROM ORGANIC WASTE. This patent discloses the method and devices for obtaining biodegradable bioplastic of bacterial origin, in addition to obtaining an amino acid-rich root biostimulant for use in agriculture, from organic waste.

Based on this development, this new invention arises which aims to obtain, from organic waste of plant or animal origin or a mixture of both, nitrogen- and/or potassium-based fertilisers for agricultural use and bacterial bioplastic.

### Description of the invention

Therefore, the object of the present invention is to provide a process and the reagents required for transforming the organic waste generated by human activity (solid and liquid organic waste from distribution centres, hotels and restaurants, solid fraction from brown containers, organic waste from agricultural activity (fruit and pruning) and from the food processing industry (orange peel, avocado skin, etc.), milk, dairy products, whey, used vegetable oils, liquid fraction digestates, etc.) in potassium- and/or nitrogen-based fertilisers as well as bacterial bioplastic.

The first step in this process is the reception of the raw materials (previously called waste) for their subsequent transformation into nitrogen- and potassium-based fertilisers and bacterial bioplastic.

The first step consists of separating the raw materials according to their origin, such that solid raw materials will be received and stored separately from liquid raw materials.

Different raw materials will be separated into 2 fractions, one solid and one liquid, according to their composition:
1.: Solid organic raw materials coming from distribution centres, hotels and restaurants, together with the organic matter from brown containers (organic fraction in selective collection), waste from pruning and from the food, agriculture and agri-food industry.
2.: Liquid organic raw materials: whey, milk and dairy products, sugary and alcoholic beverages, used vegetable oils, liquid fraction from anaerobic digestion processes, cleaning water from industrial processes for the production of musts, juices, beer, etc.

Based on the preceding 2 fractions, transformation is then performed in PHASES:

### LIQUID FRACTION

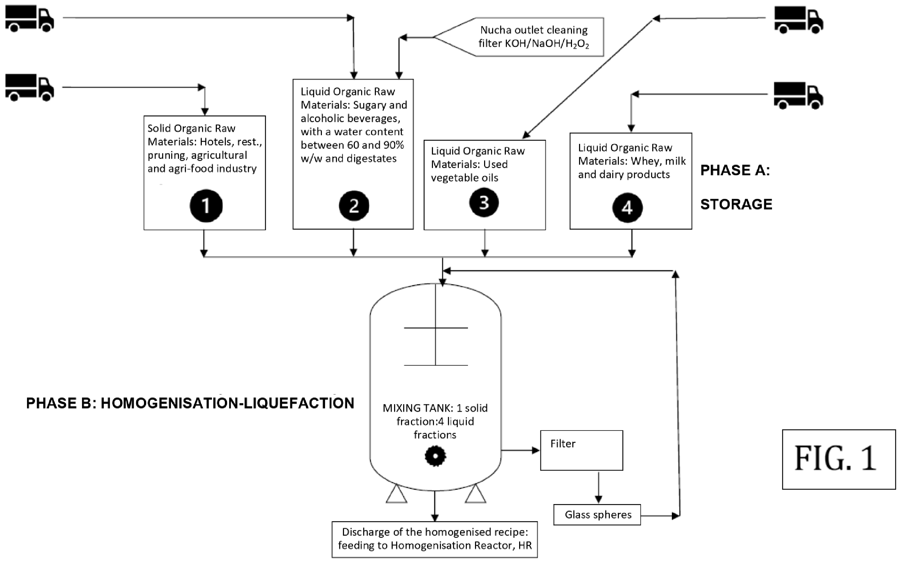

### PHASE A: STORAGE: FIG. 1

The liquid fraction is separated into three fractions:
- Sugary and alcoholic beverages, digestates from anaerobic digestion processes coming from filtration between 450 µm and 25 µm, cleaning water from industrial processes for the production of musts, juices, beer, etc. with a water content of 60 to 90% w/w
- Milk, dairy products and whey
- Used vegetable oils

Each of the three liquid fractions described above are stored separately in aseptic homogenisation tanks by means of a nitrogen (N₂) gas atmosphere, thus avoiding possible fermentation.

These raw materials will be used as dilution water for the solid raw materials in the liquefaction-homogenisation processes of PHASE B, which is described below:

### SOLID FRACTION

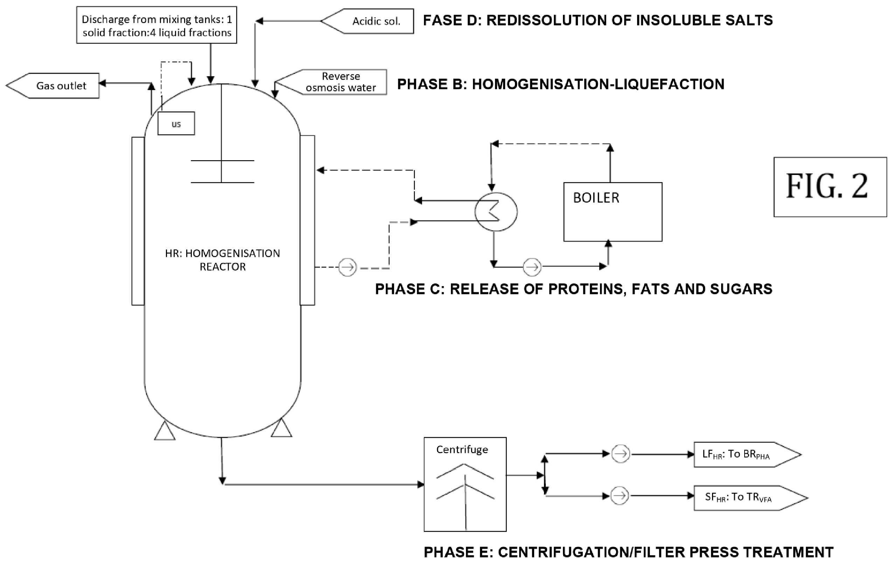

### PHASE B: HOMOGENISATION-LIQUEFACTION: Cell Lysis - FIG 1 and FIG 2.

The various types of solid organic matter will be mixed and homogenised in a mixing tank, where they will be homogenised-ground, obtaining an intimate mixture in the form of mush. Grinding the mixture will generate a mush with a variable concentration of solids with an average particle size of 50 µm and a diameter to be achieved by a two-fold effect:
- Mechanical grinding-homogenisation by a hammer mill-grinder.
- Addition of glass microspheres with a size of 100 - 1,000 µm in diameter to the mixture

To obtain the mush, the solid fraction will be mixed with each of the 3 liquid fractions described in PHASE A in a medium of high osmolarity, in a ratio of 1:1 to 1:4, solid fraction: liquid fraction, respectively. The mixture will be kept under stirring-grinding inside the mixing tank for a period of 1 hour to 12 hours, which allows for cell lysis by mechanical action. The preferred mixing-stirring-grinding time inside the mixing tank is 3 hours to 6 hours.

The recipe for the mixture of each of the three liquid fractions of PHASE A, with the solid fraction of this PHASE B, will depend in each case on the availability of the different liquid fractions. Although it does not serve as a generic rule, 1 part of used vegetable oils is normally used for every 3 parts of mixture of milk, whey or dairy products and/or sugary water/digestates.

Once the Homogenisation process is completed, the glass spheres are separated by decanting + filtration, being recovered for the next working cycle in the mixing tank.

In a second step, the thickened mush obtained above (mixture of solid fraction with liquid fraction in a ratio of 1:1 to 1:4, solid fraction:liquid fraction, respectively) in the mixing tank is sent to a Homogenisation Reactor, HR, where it will be diluted with reverse osmosis, deionised or distilled water with a conductivity < 3 µS/cm, by vigorous stirring (800 rpm ≤ Rate_{stirring} ≤ 1,400 rpm), where the liquid part of reverse osmosis/distilled/deionised water varies from 10:1 to 1:10, reverse osmosis/distilled/deionised water:solid fraction, respectively. Dilution is preferably 1:4 mush:reverse osmosis water, respectively.

Cell lysis in this PHASE B is achieved by a dual approach:
- Cell lysis by mechanical action of the grinder and the addition of glass microspheres in the phase of obtaining the thick mush mixture of solid fraction and liquid fraction in a ratio of 1:1 to 1:4, respectively.
- Cell lysis by osmotic pressure by immersing the previously obtained mush in water with very low conductivity, moving from a medium of high osmolarity to a medium of very low osmolarity

This cell lysis process allows, in a first phase, the contents of the cells to be released into the culture medium, so that now proteins, carbohydrates and lipids will be more readily available when delivered to the liquid medium. This is a very gentle method of cell lysis.

### PHASE C: RELEASE OF PROTEINS, FATS AND SUGARS: Ultrasound and/or Microwave + Direct/indirect heat. FIG 2.

After phase B, the liquid mixture of the HR in the form of a cell suspension is subjected to a mixed heating process by means of ultrasound and/or microwave radiation in the recirculation of the sample over the HR, together with direct and/or indirect heating by steam or indirect heating by thermal oil, with stirring of the sample inside the HR, as a process for releasing peptides, sugars and short-chain lipids, from cellular structures.

It will be operated at temperatures of 45°C ≤ T° ≤ 90°C and at low stirring values (150 rpm ≤ Rate_{stirring} ≤ 800 rpm) with supply of heat to the medium by steam and/or thermal oil, to release amino acids and peptides as well as sugars and oils into the culture medium, together with the application of ultrasound and/or microwaves in recirculation, which allow the fast and efficient release of the following into the culture medium:
- Low molecular weight peptides from proteins
- Sucrose and other compound sugars from glycogen, starch and cellulose
- Oils and fats contained inside membranes as energy reservoirs, as well as the cell membranes themselves.

The process for heating the sample is achieved by means of direct and/or indirect supply of steam together with US and/or microwave radiation in the recirculation of the HR and stirring inside the HR, or by means of the indirect supply of heat by thermal oil by means of an outer and/or inner jacket together with US and/or microwave radiation in the recirculation of the HR and stirring inside the HR, for a period of 1 hour to 96 hours, more preferably 24 hours to 48 hours.

Along with the release of peptides, sugars and oils and fats, the sample is sanitised by the supply of heat: steam/thermal oil and ultrasound/microwaves, together with the heating time of the mixture inside the HR.

### PHASE D: REDISSOLUTION OF INSOLUBLE SALTS/STERILISATION: Acidification of the sample. FIG 2.

The homogenised mixture of PHASE B is recirculated over the tank/reactor itself, by applying ultrasound and/or microwaves to the drive of the recirculation pump and heat directly and/or indirectly for steam or indirectly with thermal oil.

Having started the process for applying heat + ultrasound and/or microwaves, the liquid mixture, in continuous stirring, is acidified with inorganic or organic acids or a mixture thereof.

The chemical reagents used in the mixtures range from inorganic acids such as H₂SO₄, HCl, HNO₃, H₃PO₄, or mixtures thereof, together with organic acids such as lactic acid, glycolic acid, sulfamic acid, maleic acid or mixtures thereof, including acid phosphonates and polycarboxylates.

The final composition of the mixture of acids and adjuvants will depend on the composition of the mixture of solid fraction+liquid fraction and will be intended to have a dual effect:
- inhibit bacterial growth
- solubilise the precipitates of mainly insoluble calcium and magnesium salts.

The addition of the mixture of organic/inorganic acids, with stirring of the mixture, will be maintained to a stable value of 1.3 ≤ pH_{Final} ≤ 1.7, measured by a pH probe in the recirculation over the Homogenisation Reactor (HR) itself.

The mixture of solid fraction/liquid fraction at the setpoints of 1.3 ≤ pH_{Final} ≤ 1.7 allows the following:
- The addition of the mixture of acids together with stirring and monitoring of the pH value allows all the inorganic alkaline earth salts (mainly calcium and magnesium) to be re-solubilised. In this way, calcium and magnesium will be solubilised as nitrate, phosphate, sulphate, chloride, lactate, etc.
- Phosphorus, in the form of PO₄³⁻, from bones as well as from the plant part, will remain solubilised as H₃PO₄.
- Preventing the proliferation of mainly microorganisms, bacteria and fungi.
- Releasing amino acids from insoluble peptides.
- Releasing sugars from glycogen and starch.

The time that the acidified mixture is kept in recirculation-stirring inside the Homogenisation Reactor, HR, ranges from 2 hours to 96 hours. Preferably 24 hours to 48 hours.

During the time that the mixture is kept in recirculation-stirring inside the HR:
- The pH value will be maintained at a stable value of 1.3 ≤ pH_{Final} ≤ 1.7.
- Stirring (150 rpm ≤ Rate_{stirring} ≤ 800 rpm) is maintained throughout the process.
- The working temperature is maintained between 45°C ≤ T ≤ 90°C

As described, PHASES C and D are carried out at the same time, in other words, the sample starts to heat up at the same time that it is acidified, with constant stirring-recirculation over the HR.

### PHASE E: CENTRIFUGATION/FILTER PRESS TREATMENT OF THE CONTENTS IN THE REACTOR: Obtaining LF_{HR}(Liquid Fraction Homogenisation Reactor) and SF_{HR}(Solid Fraction Homogenisation Reactor) FIG 2.

After the thermal process inside the Homogenisation Reactor, HR, there is a liquid fraction and a solid fraction that have not been redissolved in the liquid fraction: Cellulose, vegetable fibre, etc.

Once the desired reaction time was reached, from 2 hours to 96 hours, the inner contents of the HR are sent to a centrifuge or a filter press, which separates the liquid fraction from the solid fraction.

The liquid fraction, LF_{HR}, will be stored in a tank from where it will be fed directly to a Biological Reactor to obtain PHA (polyhydroxyalkanoates) - BR_{PHA}, while the solid fraction, SF_{HR}, will be sent to another tank from where it will be fed to an acidogenic thermophilic reactor - TR_{VFA}.

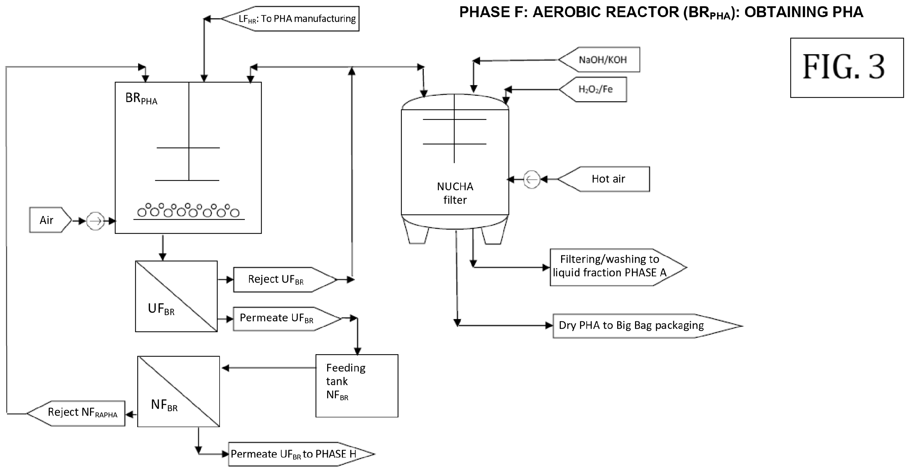

### PHASE F: AEROBIC REACTOR (BR_{PHA}): OBTAINING PHA. FIG 3.

The LF_{HR} fraction, rich in fatty acids, peptides and sugars, will feed an aerobic PHA-manufacturing reactor (BR_{PHA}), in which a PHA-overproducing bacterium has been inoculated, working under aerobic conditions and continuously.

Working under these conditions, the PHA-overproducing bacterial strain will grow under aerobic conditions, storing PHA within same on a constant basis.

Controlling the input of the LF_{HR} fraction will be done by monitoring the parameters: C.O.D. (Chemical Oxygen Demand) - D.O. (Dissolved Oxygen) and Nitrogen and Phosphorus concentrations, while bacterial growth will be mainly controlled by continuous analysis of TSS (Total Suspended Solids) which will indicate the rate of reproduction and growth of the bacteria inside the BR_{PHA}.

Phase F provides, together with the aerobic PHA-manufacturing reactor (BR_{PHA}), an ultrafiltration plant in a multitubular configuration, UF_{BR,} which allows separating the cell fraction inside the reactor, acting at the same time as a static decanter for slurry separation. This plant will operate at a molecular weight cut-off of 100,000 Daltons.

The UF_{BR} plant of the aerobic reactor will filter the contents of the aerobic reactor every time the contents of suspended solids, TSS, exceed the setpoint values. Exceeding the setpoint values indicates the presence of a sufficiently large bacterial population with sufficient PHA accumulation within same so as to carry out a PHA purge/extraction. When the TSS reading values inside the reactor exceed 10 g/l, the UF_{BR} plant will be put in operation, stopping when it reaches 5 g/l.

The UF_{BR} plant generates two streams, a permeate stream in which there are sugars/amino acids/lipids/mineral salts non-degraded in the BR_{PHA} and free of bacteria and a second fraction of solids, at a maximum concentration of solids of 25% v/v, which corresponds to the reject.

The permeate will be stored in a tank that will serve as a feeding tank to a NF (nanofiltration) plant, in a spiral configuration, NF_{BR}, for an average pore size of 250 nm (nanometres). The NF_{BR} plant will generate two streams, a permeate stream in which there will be 80% of monovalent salts (mainly K⁺:potassium and NH₄⁺:ammonium) and free of organic matter and divalent salts, and a reject in which there will be all the organic matter and divalent salts. The reject will be sent back to the aerobic PHA-manufacturing reactor, BR_{PHA}, while the permeate will be stored for the manufacture of the nitrogen- and potassium-based fertiliser together with the eluate from the adsorption columns of PHASE E.

The reject of the UF_{BR}, at a final concentration of 25% v/v, will feed a NUCHA filter in which all the solids (bacteria), together with the PHA in the form of granules, will be retained in the lower part thereof, with the aqueous stream being filtered. The aqueous stream will be added to the LF_{HR} fraction.

The solid retained in the NUCHA filter will be washed, first with a hot aqueous solution (50°C ≤ T ≤ 90°C) of 5% w/w ≤ [NaOH/KOH] ≤ 20% w/w, recirculating it over the NUCHA filter itself, in order to separate the organic matter adhered to the PHA granules. The filtered washing stream, rich in organic matter, once depleted, will be added to the liquid fraction of PHASE A corresponding to Sugary and alcoholic beverages, digestates from anaerobic digestion processes coming from filtration between 450 µm and 25 µm, cleaning water from industrial processes for the production of musts, juices, beer, etc. with a water content of 60 to 90% w/w.

The second wash, following the previous one, will be carried out with an aqueous solution of 5% w/w ≤ [H₂O₂] ≤ 30% w/w, recirculating it over the NUCHA filter itself, in order to separate the organic matter adhered to the PHA granules that has not been separated with the hot NaOH/KOH mixture. This stream will be is additivated with iron salts, FeCl₃ or Fe₂(SO₄)₃, at a dose of 10 ppm to 2,000 ppm referred to the amount of H₂O₂ added.

The filtered washing stream, once depleted, will be added to the liquid fraction of PHASE A corresponding to Sugary and alcoholic beverages, digestates from anaerobic digestion processes coming from filtration between 450 µm and 25 µm, cleaning water from industrial processes for the production of musts, juices, beer, etc. with a water content of 60 to 90% w/w.

The washing process with recirculation will be extended until the PHA granules are perfectly cleaned of organic matter. At this point, a stream of hot, dry air originating from a hot air generator will dry the PHA granules to moisture contents < 15% w/w. At this point, the NUCHA filter will be unloaded onto dry PHA collection bags.

In summary, the PHA-producing reactor has a UF_{BR} plant for separating the bacterial fraction - recovering PHA and a NF_{BR} plant to purge salts from the system, K⁺ and NH₄⁺, avoiding an increase in conductivity and NH₃ inside the Biological Reactor - BR_{PHA}.

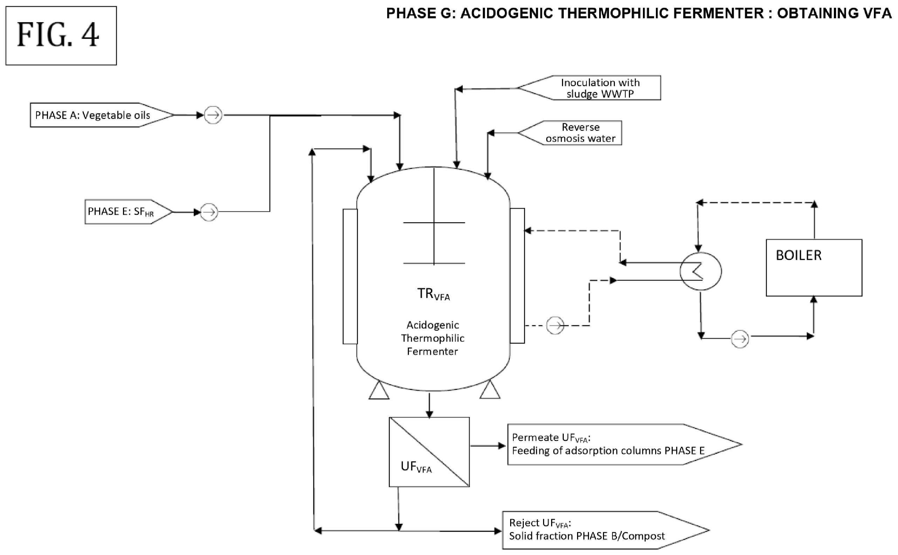

### PHASE G: ACIDOGENIC THERMOPHILIC FERMENTER: OBTAINING VFA: FIG 4

The SF_{HR} fraction obtained by the centrifuge/filter press at the outlet and discharge of the reactor, HR, will feed an acidogenic thermophilic fermenter-reactor, TR_{VFA}, in which, and under anaerobic conditions, VFA (volatile fatty acids) will be obtained from the organic matter of the SF_{HR} fraction. Together with the SF_{HR} fraction, the acidogenic thermophilic fermenter, TR_{VFA}, can be fed from the liquid fractions of PHASE A, especially from the vegetable oils.

The TR_{VFA} will be inoculated with activated sludge from WWTP (wastewater treatment plant), such that, and with the organic matter input from the SF_{HR} fraction + vegetable oils, there will be generated, by means of anaerobic acidogenic fermentation, VFA (volatile fatty acids), made up mainly of more than 90% w/w acetic acid and varying proportions of propionic acid, butyric acid, isobutyric acid and isovaleric acid.

The TR_{VFA} will operate at conditions of 5.5 ≤ pH ≤ 6.5 and at 35 ≤ T° ≤ 50, with a hydraulic retention time of 1 to 4 days.

VFA (volatile fatty acids) will be generated inside the TR_{VFA} from the continuously supplied organic matter (SF_{R} Fraction + used vegetable oils). The extraction of the VFAs generated by the fermenting bacterial strains will be done by means of a multitubular UF_{VFA} plant that allows working up to 25% v/v of solids. By means of controlling the TSS, as well as the VFA/COD/T°/pH, using probes inside the TR_{VFA}, the continuous production of the biomass, as well as VFAs (volatile fatty acids), will be monitored.

The continuous UF_{VFA} multitubular plant will filter the contents of the TR_{VFA}, generating two streams, a permeate stream which is rich in VFA and free of bacteria and a second fraction of solids at a maximum concentration of 25% v/v, which corresponds to the reject. This reject will be sent back again to the TR_{VFA}, until the TSS (total suspended solids) concentration inside the TR_{VFA} exceeds 10 g/l. At this point, the reject fraction is purged from the TR_{VFA}, either sent to the solid fraction of PHASE B or purged from the system as compostable material after drying. This process of filtration and purging to PHASE B or compost from the reject of the UF plant in multitubular configuration, UF_{VFA}, is maintained until the value of the suspended solids inside the TR_{VFA} drops below 2.5 g/l, at which time what is purged from the UF_{VFA} is sent back to the TR_{VFA} again.

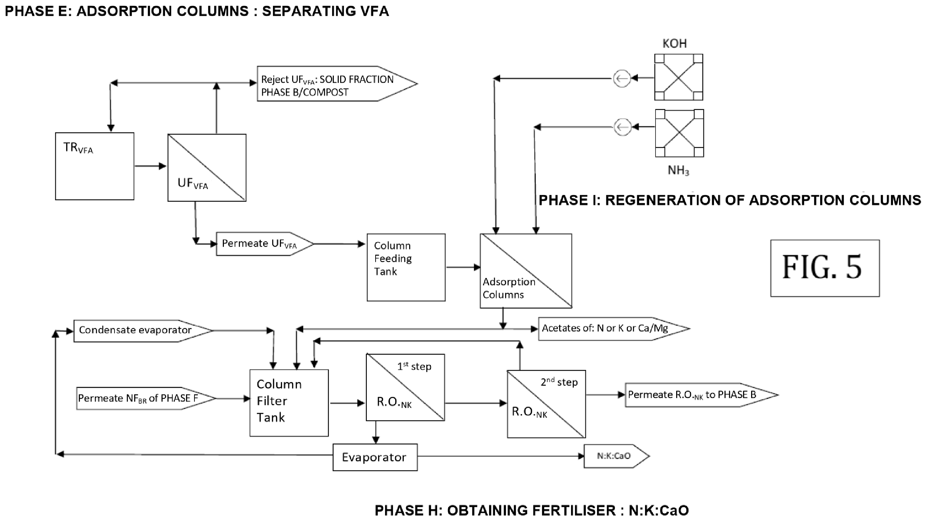

### PHASE E: ADSORPTION COLUMNS: Separating VFA : FIG 5

The permeate obtained by the UF_{VFA} membranes, equivalent to 80-90% of the input stream, will be fed to adsorption columns.

The permeate of the UF_{VFA} is a stream rich in VFA (volatile fatty acids), but it also contains the salts present in the mixed liquor of the TR_{VFA}. To separate salts from VFAs, adsorption columns will be used that retain organic matter in the form of VFA, but not ionic compounds.

The VFAs inside the TR_{VFA}, at acidic pH values, 5.5 ≤ pH ≤ 6.5, are found as acids (R-COOH) or as dissociated species, R-COO⁻, and behave as discrete molecules, but at slightly basic pH values, pH>7.0, they are transformed into their conjugated salts (R-COONa, or R-COOK or R-COONH₄) which behave like salts, not as organic matter. This duality of behaviour, covalent at acidic pH and ionic at basic pH, is the basis for the separation of VFAs from inorganic salts inside the adsorption columns.

The packing material of the adsorption columns may contain, among other materials:
Coconut shell, bituminous or pinewood activated carbon, zeolites, sepiolites, bentonites or mixtures thereof, depending on the organic matter to be retained.

In this case, mixtures of activated carbon, sodium bentonites and sodium zeolites are used.

The permeate of the UF_{VFA} has the pH value inside the TR_{VFA}, which is between 5.5 ≤ pH ≤ 6.5. At this pH value, the VFAs are found as acids:
R-COOH or R-COO⁻, where R=CH₃, CH₃-CH₂, CH₃-CH₃, CH₃-CH₂-CH₂, CH₃-CH₃-CH-CH₂, CH₃-CH₂-CH₂-CH₂ or CH₃-CH₂-CH₂-CH₂-CH₂.

When the permeate of the UF_{VFA} passes through the inside of the adsorption columns, at an acidic pH, all the VFAs are adsorbed as the soluble organic matter that they are, in the form of organic acids, at these pH values.

The adsorption columns retain the VFAs and allow a stream free of organic matter, but rich in nitrogen, potassium and phosphorus to pass through;
- Nitrate nitrogen from HNO₃ (nitric acid) used in the acidification of PHASE D
- Ammoniacal nitrogen from NH₃SO₃ (sulfamic acid) used in homogenisation - PHASE D, as well as the degradation of non-solubilised peptides in the reactor
- Potassium from mainly fruits and vegetables
- Phosphorus from bones in the form of hydroxyapatite (CaHPO₄)
- Calcium from mollusc shells, crustaceans and bones

### PHASE H: OBTAINING FERTILISER : N:K:CaO. FIG 5

This stream of eluate from the adsorption columns, free of organic matter and rich in salts, will be added to the stream rich in K⁺ and NH₄⁺, from the permeate of the NF_{BR}, feeding both streams to a double salt concentration system.

The first step will be a double-pass high-pressure R.O. plant, R.O._{NK}, where reverse osmosis water with a conductivity < 3 µS/cm will be obtained for reuse in PHASE B, and a reject, where the salt concentration will be above 70 g/l. This reject will feed an aerosol emission evaporation system, where the salts will be concentrated until an inorganic N:K:CaO fertiliser is obtained, with a final concentration of 10:2.4:2, approximately, while the water condensed in the evaporation process will be sent to the feed of the R.O._{NK} plant

### PHASE I: REGENERATION OF ADSORPTION COLUMNS : Obtaining acetates. FIG 5

The packing of the adsorption columns for VFA retention can be coconut shell, bituminous or pinewood activated carbon, zeolites, sepiolites, bentonites or mixtures thereof. In this case, preferably, a mixture of coconut shell activated carbon and sodium bentonites/zeolites will be used, where the adsorption capacity for VFAs is high adsorption, so that one kilogram of the activated carbon/bentonite/zeolite mixture adsorbs, on average, 1/3 of its weight in VFA.

Once the columns have been saturated with VFA, regeneration of the same will be carried out. For regeneration, a base, such as sodium or potassium hydroxide or ammonia or a carbonate, such as sodium or potassium carbonate or mixtures of both, will be used. Depending on the type of base used, an acetate, propionate, butyrate, isovalerate, or other will be obtained in the regeneration. Therefore, if a potassium hydroxide solution is used as a regenerant, the regeneration will yield, in the case of acetic acid, potassium acetate, according to the reaction:

CH₃-COOH + KOH → CH₃-COOK + H₂O

If a potassium carbonate solution is used as a regenerant, the regeneration will yield potassium acetate, according to the reaction:

2CH₃-COOH + K₂CO₃ → 2CH₃-COOK + CO₂ + H₂O

These reactions being a neutralisation reaction where an inorganic acid is transformed into its conjugated salt, which can now no longer be retained by adsorption inside the pores of the activated carbon/bentonite/zeolite and therefore elutes from the columns.

Therefore, the regeneration of the columns yields an eluate which is nothing more than a solution of potassium acetate (in this example), at a concentration similar to that of the regenerant used, being directly a fertiliser at the maximum concentration of 0:0:21, as a liquid fertiliser containing 0% nitrogen, 0% phosphorus and 21% w/w K₂O.

Therefore, if NH₃ is used as a regenerant, then there would be an N-based fertiliser resulting from the neutralisation reaction:

CH₃-COOH + NH₃ → CH₃-COONH₄

This being 10:0:0, at most, as another example in obtaining different fertilisers depending on the regenerant used in the desorption of the adsorption columns.

It is also possible to obtain:

2 CH₃-COOH + Ca(OH)₂ → (CH₃-COO)₂Ca + H₂O;

and/or

2 CH₃-COOH + Mg(OH)₂ → (CH₃-COO)₂Mg + H₂O

Depending on market conditions, one type of fertiliser or another, rich in potassium or nitrogen or calcium and/or magnesium will be manufactured.

## Claims

1. A process for treating organic waste of plant or animal origin or a mixture of both, for the production of bacterial bioplastic and nitrogen- and potassium-based fertilisers for agricultural use, wherein the liquid residual organic matter comes from:
a) Liquid organic raw materials with a water content of 60% w/w to 90% w/w, such as sugary and alcoholic beverages, digestates from anaerobic digestion processes coming from filtration between 450 µm and 25 µm, as well as cleaning water from industrial processes for the production of musts, juices, etc.
b) Whey, milk and dairy products,
c) used vegetable oils,
and which are stored separately in aseptic homogenisation tanks by means of a nitrogen (N₂) gas atmosphere, thus avoiding fermentation processes.

2. The process for treating organic waste of plant or animal origin or a mixture of both, for the production of bacterial bioplastic and nitrogen- and potassium-based fertilisers for agricultural use, wherein the solid residual organic matter used as raw material:
a) Comes from Distribution Centres, hotels and restaurants, together with the organic fraction in selective collection (brown container for organic waste) and organic waste from agricultural activity (fruit and pruning) and from the food processing industry (orange peel, avocado skin, etc.),
b) Is mixed with the liquid fraction (described in claim 1) in a ratio of 1:4 solid fraction: liquid fraction, respectively, and then, ground-homogenised until a mush is formed with an average particle size of 50 µm in diameter, by means of a hammer mill-crusher and addition of glass microspheres of 100 - 1,000 µm in diameter, inside a Mixing Tank for 3 hours to 6 hours,
c) The glass microspheres are recovered from the mixing tank by decanting-filtration, before emptying the contents of the mixing tank over the Homogenisation Reactor, HR,
d) The ratio of the liquid phase added to the solid fraction in the homogenisation-liquefaction inside the mixing tank is 1 part of used oils for every 3 parts of the remaining liquid fractions of PHASE A.

3. The process for treating organic waste of plant or animal origin or a mixture of both, for the production of bacterial bioplastic and nitrogen- and potassium-based fertilisers for agricultural use according to claim 2, wherein:
a) The mush inside the homogenisation tank is diluted, after grinding-homogenisation, with reverse osmosis/distilled/deionised water with an electrical conductivity, EC, less than 3 µS/cm at a ratio of 10:1 to 1:10, mush:water with a conductivity less than 3 µS/cm, respectively,
b) It is acidified, after dilution with water with an electrical conductivity less than 3 µS/cm, by adding a mixture of inorganic acids (H₂SO₄, HCl, HNO₃, H₃PO₄, or mixtures thereof) and/or organic acids (lactic acid, glycolic acid, sulfamic acid, or mixtures thereof, including acid phosphonates) to a value of 1.3 ≤ pH_{final} ≤ 1.7,
c) It is stirred vigorously (800 rpm ≤ Rate_{stirring} ≤ 1,400 rpm) throughout the process for producing mush and mixed with water with an electrical conductivity less than 3 µS/cm, to then stir slowly (150 rpm ≤ Rate_{stirring} ≤ 800 rpm), for a period from 2 hours to 96 hours, preferably from 24 hours to 48 hours, once the setpoint of 1.3 ≤ pH_{final} ≤ 1.7 has been reached,
d) It is heated, after reaching the setpoint of 1.3 ≤ pH_{final} ≤ 1.7, in a range of 45°C ≤ T° ≤ 90°C throughout the stirring process lasting from 2 hours to 96 hours.

4. The process for treating organic waste of plant or animal origin or a mixture of both, for the production of bacterial bioplastic and nitrogen- and potassium-based fertilisers for agricultural use according to claim 3, wherein the mush generated in PHASE B is heated and sanitised inside the HR:
a) Heated, by direct supply of heat by means of steam injection inside the HR, together with US and/or microwave radiation in the drive of the recirculation of the HR,
b) Or, by indirect supply of heat by means of steam/thermal oil by means of an inner and/or outer heating jacket, together with US and/or microwave radiation in the drive of the recirculation of the HR,
c) Sanitised, thanks to the working temperature inside the HR, 45°C ≤ T° ≤ 90°C throughout the process, the heating time from 2 hours to 96 hours and the acidification of the contents of the HR to values of 1.3 ≤ pH_{final} ≤ 1.7, with continuous stirring throughout the process.

5. The process for treating organic waste of plant or animal origin or a mixture of both, for the production of bacterial bioplastic and nitrogen- and potassium-based fertilisers for agricultural use according to claim 3, wherein the contents inside the HR, after the heating and sanitising process, is treated by a filter press or centrifuge for separating the solid fraction, SF_{HR}, from the liquid fraction, LF_{HR}.

6. The process for treating organic waste of plant or animal origin or a mixture of both, for the production of bacterial bioplastic and nitrogen- and potassium-based fertilisers for agricultural use according to claim 5, wherein the LF_{HR}, rich in peptides, sugars, fatty acids and mineral salts, will be sent inside the biological reactor, BR_{PHA}, where it will be fed to a PHA-overproducing bacterium, which grows inside the aerated biological reactor, BR_{PHA}, at concentrations of 5 to 10 g/l.

7. The process for treating organic waste of plant or animal origin or a mixture of both, for the production of bacterial bioplastic and nitrogen- and potassium-based fertilisers for agricultural use according to claim 6, wherein the BR_{PHA} has an ultrafiltration plant at a molecular weight cut-off of 100,000 Dalton, in multi-tubular configuration, UF_{BR}, which by filtering the mixed liquor inside the BR_{PHA} generates two streams;
• A permeate stream, stream passing through the membranes, which is rich in VFA and mineral salts, which will feed a nanofiltration plant, NF_{BR}, and
• A reject, the fraction which has not passed through the membranes, where all the bacteria is concentrated to a value of 25% v/v and which is redirected back to the BR_{PHA} when the value inside the BR_{PHA} is 5 g/l ≤ TSS ≤ 10 g/l or to a Nucha filter when the value inside the BR_{PHA} is TSS ≥ 10 g/l and up to a value of 5 g/l TSS, when again, the reject fraction of the UF_{PHA}, is directed back to BR_{PHA}.

8. The process for treating organic waste of plant or animal origin or a mixture of both, for the production of bacterial bioplastic and nitrogen- and potassium-based fertilisers for agricultural use according to claim 6, wherein BR_{PHA} has a nanofiltration (NF) plant at 250 nm pore size, in a spiral configuration, and which, being fed from the permeate of UF_{BR}, generates two streams;
• A permeate stream, stream passing through the NF membranes, with 80% of monovalent salts (mainly K⁺:potassium and NH₄⁺:ammonium) present in the permeate of the UF_{BR}, which will be stored together with the eluate from the microfiltration columns and
• Another reject stream, the fraction which has not passed through the membranes, which is rich in divalent salts and organic matter, which is sent back to the BR_{PHA}.

9. The process for treating organic waste of plant or animal origin or a mixture of both, for the production of bacterial bioplastic and nitrogen- and potassium-based fertilisers for agricultural use according to claim 6, wherein the BR_{PHA} has a NUCHA filter that receives the reject of the UF_{BR}, with a bacterial concentration of 25% v/v, which extracts PHA from the inside of the bacteria by a double washing step;
• Washing with a hot aqueous solution (50°C ≤ T ≤ 90°C) of 5% w/w ≤ [NaOH/KOH] ≤ 20% w/w, followed by a second wash with,
• A solution of 5% w/w ≤ [H₂O₂] ≤ 30% w/w, additivated with iron salts, FeCl₃ or Fe₂(SO₄)₃, at a dose of 10 ppm to 2,000 ppm referred to the amount of H₂O₂ added,
and final drying with hot, dry air generated by a low-pressure compressor, which allows PHA granules to be obtained at a dryness < 15% w/w, unloaded from the NUCHA type filter.

10. The process for treating organic waste of plant or animal origin or a mixture of both, for the production of bacterial bioplastic and nitrogen- and potassium-based fertilisers for agricultural use according to claim 9, wherein the basic washing water from the NUCHA filter (5% w/w ≤ [NaOH/KOH] ≤ 20% w/w) as well as the washing water with H₂O₂ are both sent to the liquid fraction of PHASE A corresponding to water from industrial washing processes for the food industry with a water content of 60% to 90% w/w.

11. The process for treating organic waste of plant or animal origin or a mixture of both, for the production of bacterial bioplastic and nitrogen- and potassium-based fertilisers for agricultural use according to claim 5, wherein the SF_{HR} together with part of the used vegetable oils from PHASE A will be sent inside the acidogenic thermophilic reactor, TR_{VFA}, for generating VFAs (volatile fatty acids), and wherein, operating at conditions of 5.5 ≤ pH ≤ 6.5 and at 35 ≤ T° ≤ 50, with a hydraulic retention time of 1 to 4 days, more than 90% acetic acid may be obtained as the main VFA.

12. The process for treating organic waste of plant or animal origin or a mixture of both, for the production of bacterial bioplastic and nitrogen- and potassium-based fertilisers for agricultural use according to claim 11, wherein the TR_{AGV} has an ultrafiltration plant at a molecular weight cut-off of 100,000 Dalton, in multi-tubular configuration, UF_{VFA}, which by filtering the mixed liquor inside the TR_{AGV} generates two streams;
• A permeate stream, stream passing through the membranes, which is rich in VFA and mineral salts, which will feed adsorption columns, and
• A reject, the fraction which has not passed through the membranes, where all the bacteria is concentrated to a value of 25% v/v and which is redirected back to the TR_{VFA}, when the value inside the TR_{VFA} is 5 g/l ≤ TSS ≤ 10 g/l or as purging of the system for forming compost, or to PHASE B when the value inside the TR_{VFA} is TSS ≥ 10 g/l, until the value of TSS inside the TR_{VFA} reaches 2.5g/l, at which time the reject of the UF_{VFA} is sent back to TR_{VFA} again.

13. The process for treating organic waste of plant or animal origin or a mixture of both, for the production of bacterial bioplastic and nitrogen- and potassium-based fertilisers for agricultural use according to claim 11, wherein the TR_{VFA} has a system of adsorption columns, which have adsorbent material therein such as a mixture of activated carbon together with bentonites and sodium zeolites and which are being fed from the permeate of the UF_{VFA}, and generates two streams
• An eluate stream, output stream of the nitrogen-, potassium- and phosphorus-rich adsorption columns, and
• A retentate, composed of the organic matter retained therein and which corresponds to the VFAs (volatile fatty acids) present in the permeate of the UF_{VFA}.

14. The process for treating organic waste of plant or animal origin or a mixture of both, for the production of bacterial bioplastic and nitrogen- and potassium-based fertilisers for agricultural use according to claim 13, wherein the regeneration of the adsorption column system is carried out by the addition of an aqueous solution with a base such as KOH, NH₃, CaCO₃, etc. resulting in the regeneration of an eluate composed of potassium, ammoniacal or calcium salts, respectively, of the VFAs (volatile fatty acids) retained inside the columns and the columns regenerated for the following adsorption process.

15. The process for treating organic waste of plant or animal origin or a mixture of both, for the production of bacterial bioplastic and nitrogen- and potassium-based fertilisers for agricultural use according to claim 13, wherein the composition of the regeneration of the adsorption columns yields a directly marketed fertiliser of composition 10:0:0 in the case of using NH₃ as a regenerant, 0:10:0 in the case of using KOH as a regenerant or an organic salt of calcium and/or magnesium in the case of using CaCO₃/Ca(OH)₂/MgCO₃/Mg(OH)₂.

16. The process for treating organic waste of plant or animal origin or a mixture of both, for the production of bacterial bioplastic and nitrogen- and potassium-based fertilisers for agricultural use according to claim 13, wherein the eluate of the adsorption columns, which is rich in inorganic potassium, phosphorus and nitrogen and free of organic matter, is added to the fraction of the permeate of the NF_{BR} and they are concentrated by a double concentration system:
• double-pass high-pressure R.O. Plant, R.O._{NK}
• Evaporation system.

17. The process for treating organic waste of plant or animal origin or a mixture of both, for the production of bacterial bioplastic and nitrogen- and potassium-based fertilisers for agricultural use according to claim 16, wherein the R.O._{NK} plant is a double-pass plant;
• The permeate of the first step feeds a second R.O. plant, wherein the permeate of this second step has an EC (Electrical Conductivity) ≤ 3 µS/cm, generating water that will be used as dilution water in PHASE B,
• The reject of the first step of the R.O._{NK} will feed the evaporation system,
• The reject of the second step of the R.O._{NK} will feed the first step of the R.O._{NK}.

18. The process for treating organic waste of plant or animal origin or a mixture of both, for the production of bacterial bioplastic and nitrogen- and potassium-based fertilisers for agricultural use according to claim 16, wherein the evaporation system fed by the reject of the first step of the R.O._{NK} will concentrate the salt solution by evaporation until the desired commercial N:P:K value is obtained, estimated at approximately 10:2.4:2 for organic waste of mixed plant + animal origin and a condensed water that will feed the first step of the reverse osmosis plant, R.O._{NK}.
